# EUROPEAN PATENT APPLICATION

(11) **EP 1 452 602 A1**
(43) Date of publication of application: **01.09.2004**
(21) Application number: 03472001.1
(22) Date of filing: 25.02.2003
(51) Int. Cl.: C12P 7/42, C12P 7/62

(54) **Method for production of pravastatin by fermentation**

(71) Applicant: Balkanpharma-Razgrad AD, 7200 Razgrad (BG)
(72) Inventor: Kostova, Iliana, 7200 Razgrad (BG); Ivanova, Nadegda, 7200 Razgrad (BG); Losev, Valentin, 7200 Razgrad (BG); Dimitrova, Angelina, 7200 Razgrad (BG); Vasileva, Rositza, 7200 Razgrad (BG); Todorova, Dimitra, 7200 Razgrad (BG)
(74) Representative: Valcheva, Emiliya Nikolova

(57) **Abstract**

Pravastatin is a HMG-CoA reductase inhibitor of the cholesterol biosynthesis. It exhibits well-pronounced tissue selectivity. According to the invention, the producing strain *Streptomyces roseochromogenes,* registered in the NBIMCC under Nº 8072, is cultivated under submerged conditions in a nutrient broth containing in % w/w: glucose from 1 to 3; yeast extract from 0.5 to 1.0; sodium chloride from 0.1 to 1.0; calcium carbonate from 0.05 to 0.5; glycerol from 1 to 5, under aerobic conditions at temperature of 26 - 30°C, pressure 0.5 bar and pH 7.0 - 7.5. The advantage of the method is the high degree of transformation of ML-236B (compactin) to pravastatin.

## Description

### Technical field

The invention relates to a method for production of pravastatin by fermentation, which is intended for use in the pharmaceutical industry.

### Prior art

Pravastatin has been known as an inhibitor of cholesterol biosynthesis since 1979 (Eur. J. Biochem. 1989, 184, 707-713). It is produced by hydroxylation of ML-236B (compactin) during submerged fermentation of various microorganisms from the *Gibertella, Streptomyces, Circinella, Amicolata, etc.* species. Pravastatin possesses a well-pronounced tissue selectivity, which is its advantage compared to the other HMG-CoA reduxidase inhibitors, i.e. lovastatin, simvastatin and compactin.

There is a known method, which is based on *Actinomadura* ATCC 55 678 strain, transforming ML-236B (compactin), being cultivated in a nutrient medium, containing assimilable sources of nitrogen, carbon and mineral salts. The transformation takes place at 25-27°C, with continuous stirring for 120 hours (US 5 942 423).

A disadvantage of this method is the low pravastatin yield.

A method for the production of pravastatin exists, based on the cultivation of strains of *Saccharopolyspora hirsute subsp. kobensis, Amycolata autotrophica, Streptomyces carbophylus, Streptomyces californicus, Amycolata hydrocarbon-noxydans, Amycolatopsis fastidiosa* in a fermentation medium, containing assimilable carbon sources and inorganic salts in aerobic conditions at temperature 24 - 30°C for about 144 hours (US 5 153 124; US 6 043 064).

A disadvantage of this method is the low degree of transformation of ML-236B (compactin) to pravastatin, as well as the formation of by products.

A method for the production of pravastatin is known, based on a *Streptomyces roseochromogenes* NBIMCC 1467 strain, cultivated in submerged aerobic conditions and forming pravastatin and related components.

A disadvantage of this method is the low level of transformation of ML-236B (compactin) to pravastatin, which makes it inefficient for commercial production.

### Description of the invention

The present invention is a process for the production of pravastatin from the strain *Streptomyces roseochromogenes,* registered at NBIMCC under Nº 8072, which is cultivated in submerged aerobic conditions in a nutrient medium, containing assimilable sources of nitrogen, hydrogen and inorganic salts. The fermentation is carried at temperatures of 27 - 30°C, pressure of 0.5 - 0.8 Bar and pH from 6.0 to 8.0 for 120 - 180 hours.

The strain *Streptomyces roseochromogenes* NBIMCC 8072 was obtained form *Streptomyces roseochromogenes* NBIMCC 1467 strain by means of ultraviolet irradiation and a subsequent treatment with N-methyl-N'-nitro-N-nitrosoguanidine. The resultant variant was stabilized through natural selection.

The original parent strain *Streptomyces roseochromogenes* NBIMCC 1467 and the selected improved strain were characterized using the following agar media:
1. ISP No 1
2. ISP No 2
3. ISP No 3
4. ISP No 4
5. ISP No 5
6. ISP No 7

The morphological and cultural characteristics of the two strains were determined on the 7-th day of their development and are described in Table 1.

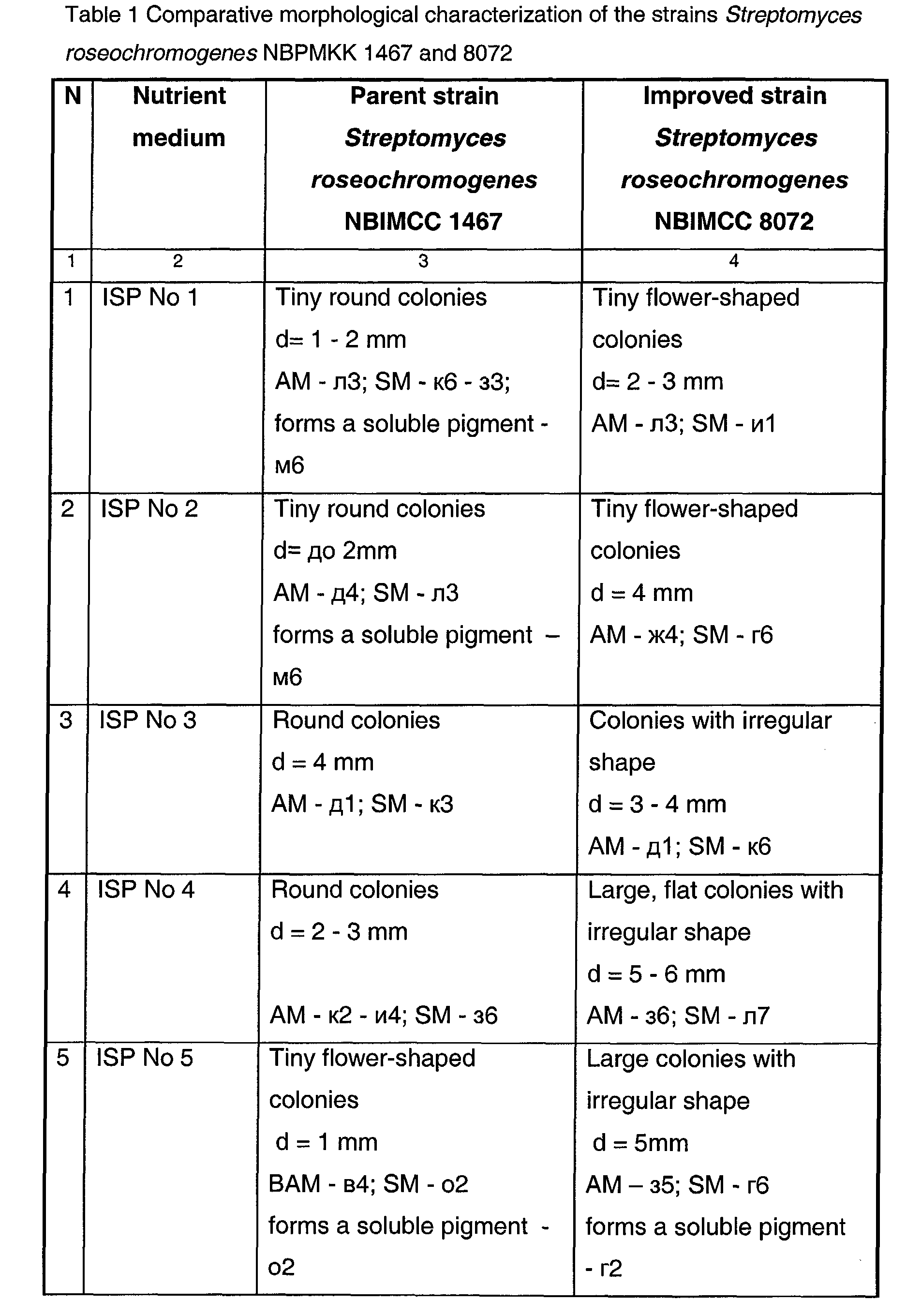

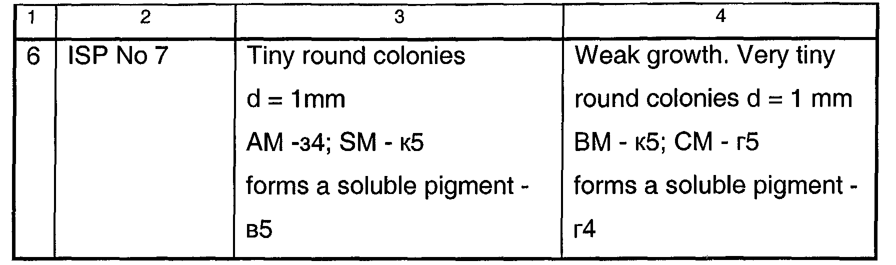

The colour of the aerial mycelium was determined after Bondartzev's scale ( 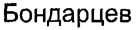 A. C., 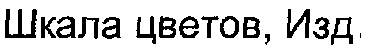 , AH CCCP, 1954).

The data on assimilation of various carbohydrate sources are presented in Table 2. The tests were performed in a medium with a single carbohydrate source in 1% concentration, containing the following in weight %: ammonium sulphate - 0.026; potassium phosphate 0.024; dipotassium phosphate - 0.56; magnesium sulphate - 0.1; salt solution∗ - 0.1; agar - 1.5; pH - 6.8±7.0.
∗Salt solution: copper sulphate - 0.63 g; ferrous sulphate - 0.11 g; manganese sulphate - 0.79 g; zinc sulphate - 0.15 g; distilled water to 100 ml. The sugars were added as sterile 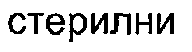 solutions at agar temperature about 60°C.

**Table2.**

| Assimilation of carbohydrate sources by the parent strain *Streptomyces roseochromogenes* NBIMCC 1467 and strain *Streptomyces roseochromogenes* NBIMCC 8072. | | | |
|---|---|---|---|
| No | Substrate | *Streptomyces roseochromogenes* NBIMCC 1467 | *Streptomyces roseochromogenes* NBIMCC 8072 |
| 1 | 2 | 3 | 4 |
| 1. | Blank | ++ | + |
| 2. | Glucose | ± | +++ |
| 3. | Fructose | ± | ++ |
| 4. | Maltose | +++ | +++ |
| 5. | Galactose | +++ | +++ |
| 6. | Sucrose | ++ | ++ |
| 7. | Lactose | ++ | +++ |
| 8. | Ribose | - | + |
| 9. | Xylose | - | ++ |
| 10. | Raffinose | ++ | +++ |
| 11. | Ramnose | + | ++ |
| 12. | Arabinose | + | + |
| 13. | Mannite | ++ | ± |
| 14. | Inosine | ++ | +++ |
| 15. | Dextrin | +++ | +++ |

The symbols in the table indicate the following: "+" - positive growth; "++" - good growth; "+++" - very good growth; "±" - marginal growth; "-" - no growth.

When the strain is stored on slant agar at temperatures up to +4°C it retains its characteristics for 1 month. For longer periods of time the strain is stored in lyophilized state.

A fresh culture of *Streptomyces roseochromogenes* NBIMCC 8072, grown in a test tube on slant agar - Gauze-1 is washed off with sterile saline solution and is homogenized. 1 ml of this suspension is used to inoculate 30 ml of nutrient broth containing the following percentage (w/w): glucose 1 - 3; yeast extract - 0.5 - 1.0; sodium chloride - 0.1 - 1.0; calcium carbonate - 0.05 - 0.5; glycerol- 1 - 5. pH is adjusted to the range 7.0 - 7.5 and the broth is sterilized at temperature 121°C for 30 min. Cultivation is performed at temperature 28°C, with continuous stirring for 24 -40 hours.

5 to 10 v/v % of the inoculum are transferred to a fermentation broth containing a carbohydrate source of 1 - 5%, sources of nitrogen 1 - 5% and mineral salts in a quantity of 0.1 - 1%. pH natural. The fermentation process is carried at temperature 26 - 30°C for 120 - 168 hours.

As sources of carbon in the fermentation broth can be used: carbohydrates, e.g. glucose, fructose or dextrin. As sources of organic nitrogen can be employed: soybean meal, soybean flour, corn steep liquor, peptone, triptone, yeast extract individually or in combinations, and as mineral salts - phosphates and chlorides of Sodium or Potassium.

A specific characteristic of the strain *Streptomyces roseochromogenes* NBIMCC 8072 is that out of all nutrient substrates it assimilates best glucose, soybean meal and peptone.

An advantage of the method is the high level of transformation of ML-236B (compactin) to pravastatin during fermentation of the specified mutant strain, as compared to the parent strain.

### Explanation of the figures

Pravastatin, obtained using the process of this invention was assayed quantitatively by a HPLC method. The test results are described as follows:

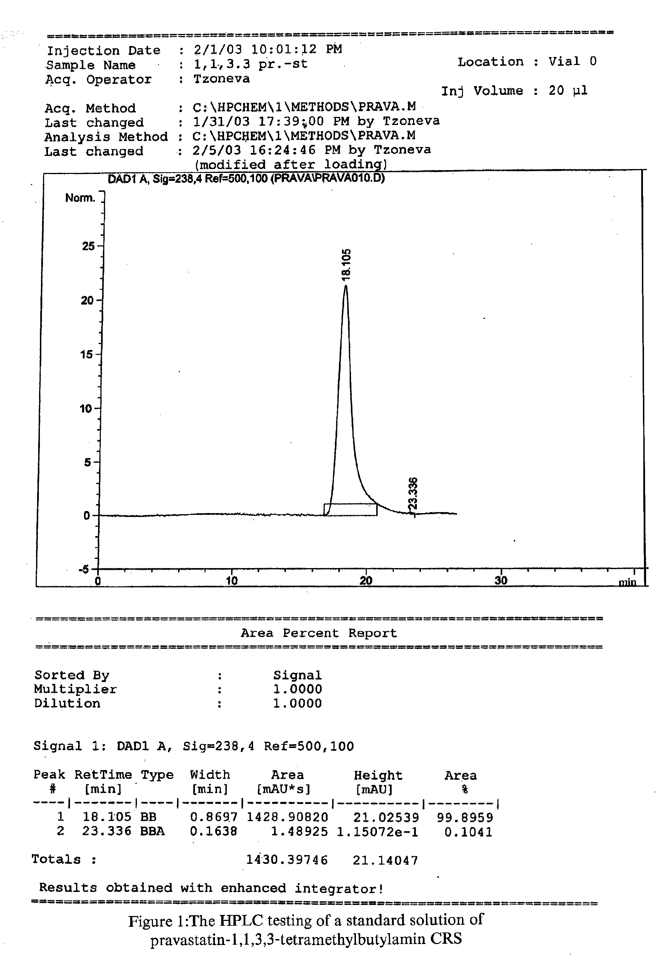

1. Figure 1. contains the results from the HPLC testing of a standard solution of pravastatin-1,1,3,3-tetramethylbutylamin CRS.

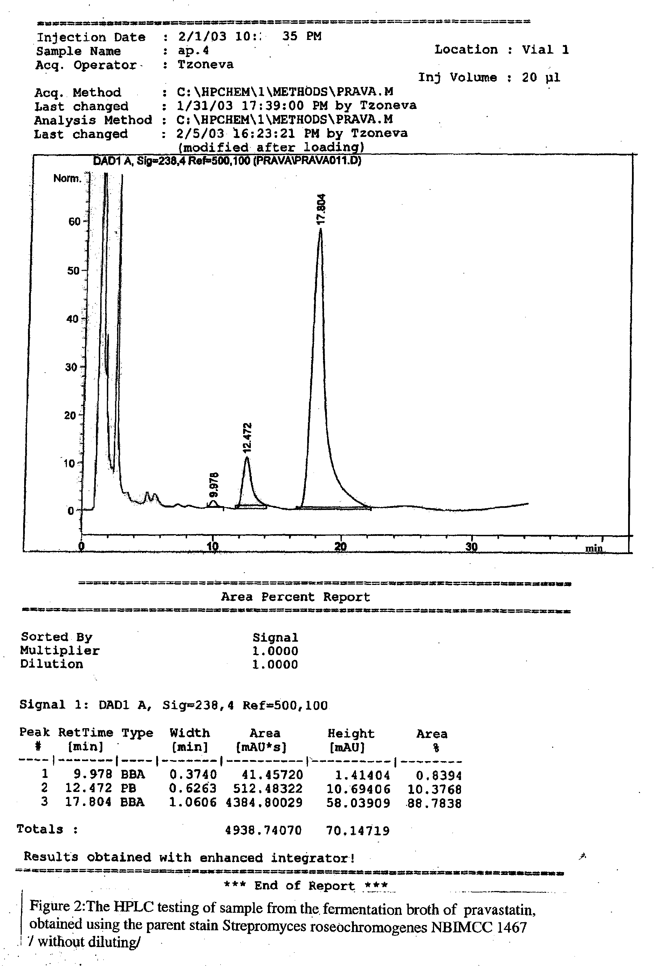

2. Figure 2. contains the results from the HPLC testing of sample from the fermentation broth of pravastatin, obtained using the parent strain.

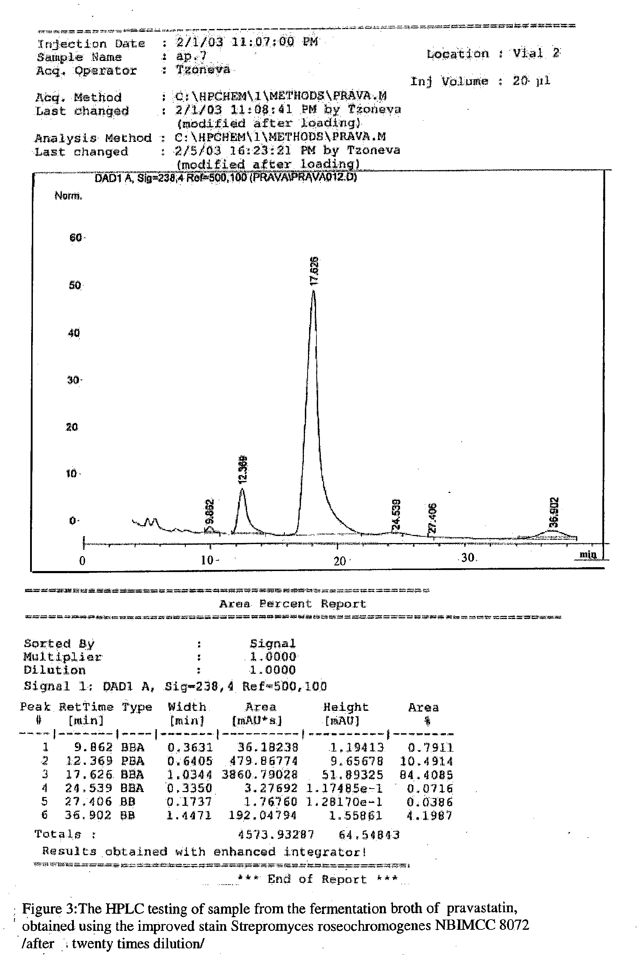

3. Figure 3. contains the results of the HPLC testing of a sample from the fermentation broth of pravastatin, obtained after the process of this invention.

### Examples:

The invention is illustrated by the following examples:

### Example 1

A fresh culture of *Streptomyces roseochromogenes* NBIMCC 8072, grown in a test tube on agar slant - Gauze-1 was washed off with sterile saline solution and was homogenized in a Potter mill. 1 ml of this suspension was used to inoculate a 300-ml Erlenmeyer flask, containing 30 ml of nutrient broth of the following composition:

| Component | Quantity (g/I) |
|---|---|
| Glucose, crystalline | 15 |
| Glycerine | 2 |
| Yeast extract | 1,5 |
| Meat peptone | 2 |
| Sodium chloride | 2 |
| Calcium carbonate | 1 |
| Tap water | To 1 I |

pH was adjusted to 7.2 ± 0.2. The broth was sterilized at 121°C for 30 min.
pH after sterilization was 7.0 ± 0.2. After cultivation at 28°C on a rotary shaker for 24 hours,
5 to 10 % (v/v) of the inoculum was transferred to a fermentation broth containing the following:

| Component | Quantity (g/l) |
|---|---|
| Glucose, crystalline | 20 |
| Yeast extract | 20 |
| Meat peptone | 10 |
| Sodium chloride | 2 |
| Calcium carbonate | 2 |
| Tap water | To 1 I |

Aliquots of 30 ml of the broth were dispensed in 300-ml Erlenmeyer flasks and the pH was adjusted to 7.4 ±0.2.
The fermentation was carried for 120 - 144 hours at 28°C on a rotary shaker with 240 rpm and 5cm deviation. Between 24 and 120 hour a 10% sterile solution of ML-236B (compactin) was added to a final concentration of 500 mg/l per day.
Upon the completion of the fermentation process, each flask was tested for quantitative content of pravastatin using an HPLC method against a reference substance. Degree of transformation - 80%.
To establish the non-biological modifications, a 10-% sterile solution of ML-236B (compactin) was added to three non-inoculated flasks to concentration of 500 mg/l per day. After the completion of the fermentation, these were tested in the same way as the inoculated flasks. No evidence was found for non-biological transformation of the compactin substrate.

### Example 2

A fresh culture of *Streptomyces roseochromogenes* NBIMCC 8072, grown in a test tube on agar slant - Gauze-1 was washed off with sterile saline solution and was homogenized in a Potter mill. 5 ml of the suspension were used to inoculate a 1000-ml Erlenmeyer flask, containing 200 ml of broth with the following composition:

| Component | Quantity (g/I) |
|---|---|
| Glucose, crystalline | 15 |
| Glycerine | 2 |
| Yeast extract | 1,5 |
| Meat peptone | 2 |
| Sodium chloride | 2 |
| Calcium carbonate | 1 |
| Tap water | To 1 I |

The pH was adjusted to 7.2 ± 0.2. The broth was sterilized at 121°C for 30 min. After the sterilization the pH was 7.0 ± 0.2. The culture was developed at 28°C on a rotary shaker at 240 rpm, with a deviation of 5 cm for 24-30 hours. The resulting material was used to inoculate a 50-litre working fermenter containing the following fermentation broth:

| Component | Quantity (g/I) |
|---|---|
| Glucose, crystalline | 20 |
| Yeast extract | 20 |
| Meat peptone | 10 |
| Sodium chloride | 2 |
| Calcium carbonate | 2 |
| Tap water | To 1 l |

The pH of the broth was adjusted to 7.2 ± 0.2 and sterilized at 121°C for 30 min. After the sterilization the pH was 7.0 ± 0.2. The inoculated broth was cultivated at temperature 28°C, aeration 1:1= volume:volume of broth , pressure 0.5 Bar for 120 - 144 hours. Between the 24-th hour from the beginning of the fermentation and the 120 hour a 10% solution of ML-236B sodium salt was fed into the culture. pH, glucose content, dissolved oxygen and the quantity of biomass were controlled in the course of the fermentation process. The transformation of ML-236B (compactin) sodium salt to pravastatin was monitored after the 48 - 50 hour.
The degree of transformation was 80-85% of the quantity of added compactin, or 6 - 9 g/l.

The content of pravastatin was determined after the HPLC method, described in the European Pharmacopoeia 2002, Suppl. 4.3 against a standard solution of Pravastatin-1,1,3,3-tetramethylbutyl amine CRS.

## Claims

1. A method for production of pravastatin by fermentation, comprising cultivation of a strain from the genus *Streptomyces roseochromogenes* in a nutrient broth, containing sources of carbon, nitrogen and mineral salts, and in particular submerged cultivation of the producing strain *Streptomyces roseochromogenes,* registered in the National Bank for Industrial Microorganisms and Cell Cultures of the Republic of Bulgaria - Nº 8072, in a nutrient broth, containing the following (in % w/w): glucose from 1 to 3; yeast extract from 0.5 to 1.0; sodium chloride from 0.1 to 1.0; calcium carbonate from 0.05 to 0.5; glycerol from 1 to 5, under aerobic conditions at temperature in the range 26 - 30°C, pressure 0.5 bar and pH 7.0 - 7.5.

## Amended claims

### Amended claims in accordance with Rule 86(2) EPC.

**1.** A method for production of pravastatin by fermentation, comprising cultivation of a strain from the genus *Streptomyces roseochromogenes* in a nutrient broth, containing sources of carbon, nitrogen and mineral salts, **characterized in that** the producing strain *Streptomyces roseochromogenes,* registered in the National Bank for Industrial Microorganisms and Cell Cultures of the Republic of Bulgaria - Nº 8072 is cultivated under submerged conditions in a nutrient broth, containing the following (in % w/w): glucose from 1 to 3; yeast extract from 0.5 to 1.0; sodium chloride from 0.1 to 1.0; calcium carbonate from 0.05 to 0.5; glycerol from 1 to 5; meat peptone from 0.1 to 2 under aerobic conditions at temperature in the range 26 - 30°C, pressure 0.5 bar and pH 7.0 - 7.5, while adding 10% solution of ML-236B sodium salt from 24^{th} hour till the end of fermentation to a final concentration of 500 mg/l per day.
